# EUROPÄISCHE PATENTANMELDUNG

(11) **EP 4 039 290 A1**
(43) Veröffentlichungstag der Anmeldung: **10.08.2022**
(21) Anmeldenummer: 22153185.8
(22) Anmeldetag: 25.01.2022
(51) Int. Cl.: A61M 5/14, A61M 5/142

(54) **GASDRUCKBETRIEBENE INFUSIONSPUMPE MIT CHIP-KAPILLARE**

(30) Priorität: 08.02.2021 DE 102021102859
(71) Anmelder: TRICUMED Medizintechnik GmbH, 24143 Kiel (DE)
(72) Erfinder: OTTO, Karl-Heinz, 24146 Kiel (DE)
(74) Vertreter: Lobemeier, Martin Landolf

(57) **Zusammenfassung**

Gasdruckbetriebene Infusionspumpe mit einem Infusatraum, einem von diesem über ein elastisches Element getrennten, mittels des elastischen Elements auf den Infusatraum wirkenden Treibmittelraum, einem Katheteranschluss und einer zwischen Infusatraum und Katheteranschluss angeordneten aus zwei miteinander verbundenen planparallel angeordneten Platten (20, 30) gebildeten, eine Drosselstrecke (40) ausbildendenen Drossel (10), wobei die Drosselstrecke (40) durch eine in wenigstens einer der beiden Platten (20) ausgebildete, von der anderen Platte (30) verschlossenen Vertiefung gebildet ist, die an ihrem einen Ende einen mit dem Infusatraum kommunizierenden Einlass und an ihrem anderen Ende einen mit dem Katheteranschluss kommunizierenden Auslass aufweist, dadurch gekennzeichnet, dass wenigstens eine der beiden Platten (20, 30) aus Korund besteht.

## Beschreibung

Die Erfindung betrifft eine gasdruckbetriebene Infusionspumpe mit einem Infusatraum, einem von diesem über ein elastisches Element getrennten, mittels des elastischen Elements auf den Infusatraum wirkenden Treibmittelraum, einem Katheteranschluss und einer zwischen Infusatraum und Katheteranschluss angeordneten aus zwei miteinander verbundenen planparallel angeordneten Platten gebildeten, eine Drosselstrecke ausbildendenen Drossel, wobei die Drosselstrecke durch eine in wenigstens einer der beiden Platten ausgebildete, von der anderen Platte verschlossenen Vertiefung gebildet ist, die an ihrem einen Ende einen mit dem Infusatraum kommunizierenden Einlass und an ihrem anderen Ende einen mit dem Katheteranschluss kommunizierenden Auslass aufweist.

Als gasdruckbetriebene Infusionspumpe ausgebildete implantierbare Infusionspumpen zur konstanten intrathekalen Langzeit-Medikation sind hinlänglich bekannt. Sie werden insbesondere in der Schmerz- und Spastiktherapie verwendet und ermöglichen dem Patienten ein weitgehend normales und beschwerdefreies Leben. Speziell werden durch eine gleichmäßige und geringe Medikamentenabgabe von ca. 1/100 der ansonsten üblichen bzw. notwendigen oralen Dosis die Nebenwirkungen des jeweils verwendeten Medikaments drastisch verringert.

Um eine kontinuierlich gleichmäßige Medikamentenabgabe zu ermöglichen, ist zwischen dem Medikamentenreservoir, also dem Infusatraum, und dem Pumpenauslass, also dem Katheteranschluss, eine Drossel mit einer vorbestimmten Drosselstrecke vorgesehen, die die Flussrate und zusammen mit dem Reservoirvolumen das Auffüllintervall der Infusionspumpe bestimmt.

Ursprünglich wurden als Drossel sich linear erstreckende, mit einer Außenbeschichtung versehene Glaskapillare mit einem vorbestimmten Durchmesser verwendet, die zu einer Rolle, auch "coil" genannt, aufgewickelt wurden. Der Nachteil dieser Art Drossel besteht darin, dass der Durchmesser der Kapillare über deren Länge, also über die Drosselstrecke erheblichen Schwankungen unterliegt und damit einen hohen Ausschuss an Kapillarmaterial in der Größenordnung von 10-15 % produziert. Der Arbeits- und Materialaufwand bei der Herstellung implantierbarer Infusionspumpen ist also aufgrund der Auswahl geeigneter Kapillar-Abschnitte erhöht. Darüber hinaus führen beim Aufwickeln entstehende Haarrisse nach unbestimmter Zeit zum Bruch der Kapillare, sodass diese tatsächlich eine nur begrenzte Haltbarkeit hat.

Abhilfe schaffen Chip-Kapillare. Diese sehen eine Drossel mit einer von zwei miteinander verbundenen planparallel angeordneten Platten ausgebildeten Drosselstrecke vor, wobei die Drosselstrecke durch eine in wenigstens einer der beiden Platten ausgebildete, von der anderen Platte verschlossenen Vertiefung gebildet ist, die an ihrem einen Ende einen mit dem Infusatraum kommunizierenden Einlass und an ihrem anderen Ende einen mit dem Katheteranschluss kommunizierenden Auslass aufweist. Bei der Chip-Kapillare ist die die Vertiefungen aufweisende Platte aus Silizium gebildet und die die Vertiefungen abdeckende Kapillare ist aus Glas gebildet.

Wenngleich die Chip-Kapillare im Hinblick auf eine konstante Fertigung mit geringen Toleranzen unter Reinraumbedingungen und die Miniaturisierung der Drossel und damit der Pumpe als solche durchaus Vorteile besitzt, weist eine als Chip-Kapillare ausgestaltete miniaturisierte Drossel auch Nachteile auf. Insbesondere ist die Verwendung von Siliziumplatten im Hinblick auf deren chemische Beständigkeit innerhalb eines neutralen pH-Bereichs problematisch, sodass speziell Medikamente mit einem basischen pH-Wert die die Drosselstrecke ausbildenden feinen Strukturen in der Siliziumplatte zerstören und so zu einem Kurzschluss zwischen Medikamentenkammer und Auslass führen können. Nicht unerhebliche Bedeutung hat dabei auch, dass entlang der Kapillare Oberflächenmaterial abgetragen und dadurch der Querschnitt der Kapillare vergrößert werden kann, sodass selbst kleinste Änderungen der Durchflussmenge zu einer Überdosierung führen können.

Aufgabe der Erfindung ist es daher, eine implantierbare gasdruckbetriebene Infusionspumpe zu schaffen, die für Medikamente unabhängig von deren pH-Wert geeignet ist, sodass Medikamente unabhängig von deren pH-Wert infundiert werden können, ohne die Infusionspumpe bzw. deren Drossel zu beschädigen oder zu zerstören.

Diese Aufgabe wird erfindungsgemäß durch die gasdruckbetriebene Infusionspumpe mit den Merkmalen von Anspruch 1 gelöst. Die Unteransprüche geben vorteilhafte Ausgestaltungen der Erfindung wieder.

Grundgedanke der Erfindung ist es, eine gasdruckbetriebene Infusionspumpe mit einer Drossel zu schaffen, die präzise herzustellen und gegenüber der chemischen Zusammensetzung der durch die Drossel geführten Chemikalien beständig ist. Die Verwendung des Minerals Korund zur Ausbildung der Drosselstrecke bietet dabei den Vorteil eines inerten Stoffs, der mit den von der Infusionspumpe abgegebenen Medikamenten keine physikochemischen Wechselwirkungen eingeht. Dieses gewährleistet einerseits die chemische Integrität des verabreichten Medikaments und andererseits die Langlebigkeit der Drosselstrecke mit unverändertem Querschnitt.

Erfindungsgemäß wird also gasdruckbetriebene Infusionspumpe mit einem Infusatraum, einem von diesem über ein elastisches Element getrennten, mittels des elastischen Elements auf den Infusatraum wirkenden Treibmittelraum, einem Katheteranschluss und einer zwischen Infusatraum und Katheteranschluss angeordneten aus zwei miteinander verbundenen planparallel angeordneten Platten gebildeten, eine Drosselstrecke ausbildendenen Drossel, wobei die Drosselstrecke durch eine in wenigstens einer der beiden Platten ausgebildete, von der anderen Platte verschlossenen Vertiefung gebildet ist, die an ihrem einen Ende einen mit dem Infusatraum kommunizierenden Einlass und an ihrem anderen Ende einen mit dem Katheteranschluss kommunizierenden Auslass aufweist, wobei wenigstens eine der beiden Platten aus Korund besteht. Der zu diesem Zweck verwendete Korund kann natürlichen Ursprungs oder synthetisch produziert sein.

Nach einer bevorzugten Ausgestaltung weist die aus Korund bestehende Platte die die Drosselstrecke bildende Vertiefung auf, wobei die Vertiefung besonders bevorzugt durch Ätzen oder durch Lasern gebildet ist.

Nach einer weiteren bevorzugten Ausgestaltung sind beide Platten der Drossel aus Korund hergestellt.

Unabhängig davon, ob eine oder beide Platten der Chip-Kapillare aus Korund hergestellt sind, ist das für die Platte(n) verwendete Korund bevorzugt Saphir, also eine Varietät des Korund, die im trigonalen Kristallsystem mit der chemischen Zusammensetzung Al₂O₃ kristallisiert und überwiegend doppelseitig zugespitzte, tonnenförmige, sechsseitige pyramidale und prismatische Kristalle entwickelt.

Weiter ist bevorzugt vorgesehen, dass die Drosselstrecke als archimedische Spirale ausgebildet ist. Dadurch kann auf geringem Raum eine lange Drosselstrecke verwirklicht werden, wobei zusätzlich zur Verwendung von Korund aufgrund der laminaren Strömungsverhältnisse in der archimedischen Spirale verhindert wird, dass sich Partikel innerhalb der Spirale absetzen können.

Der Einlass und/oder der Auslass erstrecken sich nach einer weiteren bevorzugten Ausgestaltung durch die eine Platte und/oder die andere Platte, wobei höchst bevorzugt der Einlass in der einen Platte und der Auslass in der anderen Platte angeordnet ist.

Durch Verwendung einer besonders bevorzugt ausgestalteten Drossel nach der Erfindung, bei der insbesondere beide Platten aus Korund gefertigt sind, ist es möglich Medikamente mit unterschiedlicher Zusammensetzung zu fördern, ohne dass es zu einer Alterung der Drossel und damit zu Abweichungen der Förderrate kommt.

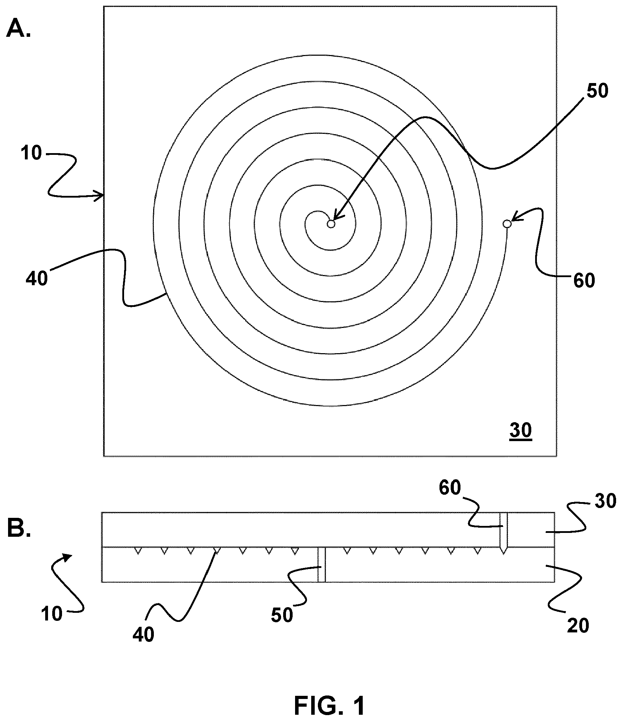

Die Erfindung wird im Folgenden anhand eines in Fig. 1 dargestellten, besonders bevorzugt ausgestalteten Ausführungsbeispiels näher erläutert, bei dem ausschließlich die Drossel einer erfindungsgemäß ausgestalteten Infusionspumpe, nicht aber die Infusionspumpe selbst gezeigt ist. Dabei zeigen Fig. 1A eine Draufsicht auf die besonders bevorzugt ausgestaltete Drossel und Fig 1B einen Querschnitt durch das Zentrum der Drossel.

Die Drossel 10 weist zwei miteinander verbundene planparallel angeordnete Platten 20, 30 auf, die eine Drosselstrecke 40 ausbilden. Im gezeigten Beispiel ist in Draufsicht zu erkennen, dass die Drosselstrecke 40 in der Form einer archimedischen Spirale ausgebildet ist. Der Einlass 50 der Drosselstrecke 40 befindet sich dabei im Zentrum der archimedischen Spirale und der Auslass 60 der Drosselstrecke 40 am äußeren Ende der archimedischen Spirale, wobei - wie Fig. 1B zeigt - der Einlass 50 in der einen Platte 20 und der Auslass 60 in der anderen Platte 30 angeordnet sind.

Die Drosselstrecke 40 selbst wird durch in der einen Platte 20 vorgesehene Vertiefungen ausgebildet, die von der anderen Platte 30 verschlossen sind. Lediglich der Einlass 50 und der Auslass 60 kommunizieren mit den als Drosselstrecke 40 ausgebildeten Vertiefungen, wobei die beiden aus Korund bestehenden Platten 20, 30 im Übrigen miteinander fluiddicht gebondet sind. Insbesondere bestehen die beiden Platten 20, 30 aus demselben Material, beispielsweise Saphir. Ein Austreten eines in die Drossel 10 eingeführten Medikaments ist also nur durch den Auslass 60 möglich.

Die die Drosselstrecke ausbildende Vertiefung ist bevorzugt durch Ätzen oder durch Lasern gebildet. Die Verwendung von ausschließlich Korund als Plattenmaterial ermöglicht das Durchleiten von Medikamenten jedes pH-Werts, ohne dass die die Struktur der Drossel 10, insbesondere der Drosselstrecke 40 beschädigt wird. Dabei verhindert auch die die Ausprägung der Drosselstrecke 40 als archimedische Spirale ein Absetzen von Partikeln innerhalb der Drosselstrecke 40, sodass eine starke Miniaturisierung der Drossel als solche möglich ist.

Innerhalb einer Infusionspumpe sorgt - wie bekannt - ein der Drossel 10 vorgeschalteter Filter (nicht dargestellt) dafür, dass der Einlass 50 der Drossel 10 nicht durch Partikel versperrt und die Pumpenfunktion so aufrechterhalten wird.

## Patentansprüche

1. Gasdruckbetriebene Infusionspumpe mit einem Infusatraum, einem von diesem über ein elastisches Element getrennten, mittels des elastischen Elements auf den Infusatraum wirkenden Treibmittelraum, einem Katheteranschluss und einer zwischen Infusatraum und Katheteranschluss angeordneten aus zwei miteinander verbundenen planparallel angeordneten Platten (20, 30) gebildeten, eine Drosselstrecke (40) ausbildendenen Drossel (10), wobei die Drosselstrecke (40) durch eine in wenigstens einer der beiden Platten (20) ausgebildete, von der anderen Platte (30) verschlossenen Vertiefung gebildet ist, die an ihrem einen Ende einen mit dem Infusatraum kommunizierenden Einlass und an ihrem anderen Ende einen mit dem Katheteranschluss kommunizierenden Auslass aufweist,
**dadurch gekennzeichnet, dass**
wenigstens eine der beiden Platten (20, 30) aus Korund besteht.

2. Gasdruckbetriebene Infusionspumpe nach Anspruch 1, **dadurch gekennzeichnet, dass** die aus Korund bestehende Platte (20) die die Drosselstrecke (40) bildende Vertiefung aufweist.

3. Gasdruckbetriebene Infusionspumpe nach einem der vorhergehenden Ansprüche, **dadurch gekennzeichnet, dass** wenigstens eine der beiden Platten (20, 30) aus Saphir besteht.

4. Gasdruckbetriebene Infusionspumpe nach einem der vorhergehenden Ansprüche, **dadurch gekennzeichnet, dass** die Drosselstrecke (40) als archimedische Spirale ausgebildet ist.

5. Gasdruckbetriebene Infusionspumpe nach einem der vorhergehenden Ansprüche, **dadurch gekennzeichnet, dass** sich der Einlass (50) und/oder der Auslass (60) durch die eine Platte (20, 30) und/oder die andere Platte (20, 30) erstrecken.

6. Gasdruckbetriebene Infusionspumpe nach einem der vorhergehenden Ansprüche, **dadurch gekennzeichnet, dass** der Einlass (50) in der einen Platte (20) und der Auslass (60) in der anderen Platte (30) angeordnet ist.
